(19) **Europäisches Patentamt / European Patent Office / Office européen des brevets**

(11) **EP 3 511 009 A1**

(12) **EUROPEAN PATENT APPLICATION**

(43) Date of publication:
**17.07.2019 Bulletin 2019/29**

(21) Application number: **18180133.3**

(22) Date of filing: **27.06.2018**

(51) Int Cl.:
**A61K 33/38** (2006.01)   **A61K 9/00** (2006.01)
**A61K 47/26** (2006.01)   **A61P 35/00** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.01.2018   CN 201810036238**

(71) Applicant: **Shenzhen AMCAN Medical
Biotechnology Limited
Shenzhen, Guangdong (CN)**

(72) Inventors:
• **SITU, Weiyi
Shenzhen,
Guangdong (CN)**
• **XIE, Hui
Shenzhen, Guangdong (CN)**
• **WANG, Yang
Shenzhen, Guangdong (CN)**
• **FAN, Rong
Shenzhen, Guangdong (CN)**

(74) Representative: **de Arpe Fernandez, Manuel
Arpe Patentes y Marcas, S.L.P.
C/Proción, 7 Edif. América II
Portal 2, 1° C
28023 Madrid-Aravaca (ES)**

(54) **ANGSTROM SILVER INJECTION FOR CANCER INHIBITION, PREPARATION METHOD AND APPLICATION THEREOF**

(57)    The present disclosure relates to an angstrom silver injection for a cancer inhibition, a preparation method and an application. A composition of the angstrom silver injection includes: angstrom-level elementary substance silver powder with a concentration between 0.005 g/L and 0.500 g/L; medicinal anhydrous fructose with a concentration between 0.5 g/L and 1.0 g/L; sterile distilled water with a concentration between 998.500 g/L and 999.495 g/L. Tumor cell membrane surface has higher negative charge, and the increase of the negative charge on the membrane surface relates to acid mucopolysaccharide, DNA, and side chain charge of protein, especially to the increase or exposure of sialic acid. Angstrom silver and silver ions have positive charge to combine with the protein and nucleic acid molecule with negative charge inside and outside cells for interfering with synthesis of the protein and replication and transcription of the DNA to destroy normal life activity of cancerous cells.

FIG. 1

**Description**

BACKGROUND

1. Technical Field

The present disclosure generally relates to angstrom silver injections field, and especially relates to an angstrom silver injection for cancer inhibition, a preparation method and an application thereof.

2. Description of Related Art

Cancer is becoming the second killer of human health around the world, thereby its treatment is also becoming an important global issue. In 2015, China added 4,292 million cancer patients. That is to say, there are added more than 8 new cancer patients per minute. Among them, liver cancer, lung cancer and pancreatic cancer are a higher incidence of cancers, at the same time, 2.814 million cancer patients has been dead. At present, a conventional treatment is generally used therapies such as a surgery, a radiotherapy, a chemotherapy and a traditional Chinese medicine to combat cancer. With the development of modern molecular biology technology and genetic engineering technology, a biological treatment method has become a fourth treatment mode. However, the above several kinds of treatment methods have their limitations, especially most treatment methods, such as the chemotherapy and the surgery, can seriously affect the quality of life of a cancer patient. Thus, the treatment of the cancer still has a long way to go.

SUMMARY

The technical problems to be solved: in view of the shortcomings of the related art, the present disclosure relates to an angstrom silver injection for a cancer inhibition, a preparation method and an application thereof.
The technical solution adopted for solving technical problems of the present disclosure is:
an angstrom silver injection for a cancer inhibition with its composition of the angstrom silver injection includes the following: angstrom-level elementary substance silver powder with a concentration between 0.005 g/L and 0.500 g/L, medicinal anhydrous fructose with a concentration between 0.5 g/L and 1.0 g/L, and sterile distilled water with a concentration between 998.500 g/L and 999.495 g/L.
Wherein the purity of silver in the angstrom-level elementary substance silver powder is not less than 99.993%, and a particle size of silver particle is between 1 Ang and 25 Ang.
Wherein the shape of the silver particle is spherical or oval.
In another aspect, a preparation method of an angstrom silver injection for a cancer inhibition according to an exemplary embodiment of the present disclosure includes the following steps: S1, Obtaining high purity angstrom silver solution with a concentration between 0.005 g/L and 0.500 g/L by adding angstrom-level elementary substance silver powder with its particle size between 1 Ang and 25 Ang to sterile distilled water; S2, dispersing and then atomizing the angstrom silver solution by means of an ultrasonic device, and finally collecting it; S3, adding fructose in the collected angstrom silver solution, and dispersing the angstrom silver solution by the ultrasonic device once again, and finally left it standing; S4, Obtaining the angstrom silver injection by collecting atomization substance after the static angstrom silver solution atomized by means of the ultrasonic device.
Wherein the step of dispersing and then atomizing the angstrom silver solution by means of an ultrasonic device, and finally collecting it includes that the angstrom silver solution is dispersed by an ultrasonic dispersion device with an ultrasonic power of 3 KW, a frequency between 30 KHZ and 40 KHZ and a dispersion time between 30 s and 60 s; and then the angstrom silver solution is atomized by an ultrasonic atomization device with an ultrasonic power of 7.5 KW, a frequency between 15 KHZ and 20 KHZ and an atomization environment temperature in 2±5 degree.
Wherein the step of adding fructose in the collected angstrom silver solution, and dispersing the angstrom silver solution by the ultrasonic device once again, and finally left it standing includes that a concentration of the fructose is between 0.5 g/L and 1.0 g/L. When the ultrasonic dispersion is repeated, the ultrasonic power of the ultrasonic dispersion device is 0.5 KW, the frequency is 110-150 KHZ, the dispersion time is 10-15 s, and a static time is 15-30 minutes.
Wherein the step of obtaining the angstrom silver injection by collecting atomization substance after the static angstrom silver solution atomized by means of the ultrasonic device includes that the ultrasonic power of the ultrasonic atomization device is 7.5 KW, the frequency is 15-20 KHZ and the atomization environment temperature in 2±5 degree.
In a third aspect, an application of an angstrom silver injection according to an exemplary embodiment of the present disclosure includes the angstrom silver injection provided for a cancer inhibition. The angstrom silver injection with its composition of the angstrom silver injection includes the following: angstrom-level elementary substance silver powder with a concentration between 0.005 g/L and 0.500 g/L, medicinal anhydrous fructose with a concentration between 0.5 g/L and 1.0 g/L, and sterile distilled water with a concentration between 998.500 g/L and 999.495 g/L.

Wherein the cancer is selected from lung cancer, liver cancer, pancreatic cancer, prostate cancer and leukemia.

The present disclosure provides the advantages as below.

A membrane surface of a tumor cell has higher negative charge, and the increase of the negative charge on the membrane surface is related to acid mucopolysaccharide, DNA, and side chain charges of protein, especially related to the increase or exposure of sialic acid. Angstrom silver and silver ions have positive charge so that they both can be combined with the protein and nucleic acid molecules with negative charge inside and outside the cell for interfering with synthesis of the protein and replication and transcription of the DNA, thereby destroying normal life activity of cancer cells.

## BRIEF DESCRIPTION OF THE DRAWINGS

FIG. 1 is a schematic view of the best group of efficacy of the angstrom silver injection for cancer inhibition in accordance with a second embodiment.

FIG. 2 is similar to FIG. 1, but shown the worst group of efficacy.

FIG. 3 is a schematic view of the angstrom silver injection for cancer inhibition added as a drug efficacy of FIG. 1.

FIG. 4A and FIG. 4B are schematic views of the best group of efficacy of the angstrom silver injection for cancer inhibition in accordance with a third embodiment.

FIG. 5A and FIG. 5B are similar to FIG. 4A and FIG. 4B, but shown the worst group of efficacy.

FIG. 6A and FIG. 6B are schematic views of the best group of efficacy of the angstrom silver injection for cancer inhibition in accordance with a fourth embodiment.

FIG. 7A and FIG. 7B are similar to FIG. 6A and FIG. 6B, but shown the worst group of efficacy.

FIG. 8 is a schematic view of efficacy of a new drug of the present disclosure.

FIG. 9A and FIG. 9B are schematic views of the best group of efficacy of the angstrom silver injection for cancer inhibition in accordance with a fifth embodiment.

FIG. 10A and FIG. 10B are similar to FIG. 9A and FIG. 9B, but shown the worst group of efficacy.

FIG. 11 is a schematic view of a detection of cell proliferation activity of cancer cells interposed by the angstrom silver injection with marked concentration after 24 hours.

FIG. 12 is similar to FIG. 11, but shown a schematic view of the detection of cell proliferation activity after 48 hours.

FIG. 13 is a schematic view of an HPAC tumor subcutaneously removed from a nude mouse in accordance with a seventh embodiment.

FIG. 14 is a schematic view of a tumor mass of the HPAC tumor subcutaneously removed from the nude mouse of FIG. 13.

FIG. 15 is a schematic view of a tumor volume of the HPAC tumor subcutaneously removed from the nude mouse of FIG. 13.

## DETAILED DESCRIPTION

The disclosure is illustrated by way of example and not by way of limitation in the figures of the accompanying drawings, in which like reference numerals indicate similar elements.

Angstrom silver material is a new kind of angstrom material developed in recent years. It is a kind of metal powder with an angstrom grade that is made from elemental metallic silver and other silver-containing compounds using a physical or chemical process to produce, while its particle size is mostly less than 100 Ang so that it is named angstrom silver. A preparation process roughly includes the following: inputting a metal wire below a certain size into an explosion chamber and blasting it by a high pressure ionization, and then gathering metal vapor by ventilation; and sending it to a buffer by a ventilation system for cooling, demagnetization, ultrasonic dispersion, separation and classification by a whirlwind way, and then collecting powder to a storage tank; finally obtaining a finished product by adding inert gas in the storage tank for passivating it.

The angstrom silver injection for a cancer inhibition of the present disclosure includes the following elements with their corresponding concentrations: angstrom-level elementary substance silver powder with a concentration between 0.005 g/L and 0.500 g/L, medicinal anhydrous fructose with a concentration between 0.5 g/L and 1.0 g/L, and sterile distilled water with a concentration between 998.500 g/L and 999.495 g/L.

Furthermore, purity of silver in the angstrom-level elementary substance silver powder is not less than 99.993%, and a particle size of silver particle is between 1 Ang and 25 Ang. The shape of silver particle is spherical or oval.

The optimum preparation method of the angstrom silver injection for the cancer inhibition includes the following:

(1) Obtaining high purity angstrom silver solution with a concentration between 0.005 g/L and 0.500 g/L by adding angstrom-level silver powder, with its particle size between 1 Ang and 25 Ang, to water;

(2) Dispersing the angstrom silver solution by means of an ultrasonic dispersion device with an ultrasonic power of 3 KW, a frequency between 30 KHZ and 40 KHZ and a dispersion time between 30 seconds and 60 seconds;

(3) Atomizing the angstrom silver solution treated with the step (2) by an ultrasonic atomization device with an ultrasonic power of 7.5 KW, a frequency between 15 KHZ and 20 KHZ and an atomization environment temperature in 2±5 degree;

(4) Adding fructose in the collected angstrom silver solution treated with the step (3), with a concentration of the fructose between 0.5 g/L and 1.0 g/L; and ultrasonic dispersing the angstrom silver solution by the ultrasonic dispersion device once again, at this time, the ultrasonic power of the ultrasonic dispersion device is 0.5 KW, the frequency is 110-150 KHZ, the dispersion time is 10-15 seconds;

(5) Leaving the silver solution treated with the step (4) to stand for 15-30 minutes;

(6) Atomizing the angstrom silver solution treated with the step (5) by the ultrasonic atomization device once again, at this time, the ultrasonic power is 7.5 KW, the frequency is between 15 KHZ and 20 KHZ and the atomization environment temperature is in 2±5 degree;

(7) Obtaining the angstrom silver injection by collecting atomization substance treated with the step (6).

An anti-cancer mechanism of the angstrom silver injection can be divided into three parts: 1. The angstrom silver can be contacted with surface protein of cancerous cell or directly through a cell membrane into a cell body to mediate the rupture of the cell membrane, thereby leading to release of content of the cancerous cell and induce death of the cancerous cell. 2. An angstrom silver surface can continuously release a silver ion so that a higher concentration of the silver ion in a local area is formed, which can lead to a stronger anti-cancer effect. 3. Both the angstrom silver and the silver ion released by the angstrom silver can induce intracellular ROS production, which can lead to DNA damage and protein oxidation in the cancer cell.

Studies have shown that the angstrom silver in the injection can be directly contacted with all kinds of cancer cell surfaces. This is because a tumor cell membrane surface has higher negative charge, and the increase of the negative charge on the membrane surface relates to acid mucopolysaccharide, DNA, and side chain charge of protein, especially to the increase or exposure of sialic acid. Both the angstrom silver and the silver ion have positive charge to combine with the protein and nucleic acid molecule with negative charge inside and outside the cell for interfering with synthesis of the protein and replication and transcription of the DNA to destroy normal life activity of the cancerous cell. The strength of the anti-cancer effect of the angstrom silver depends on a size of its particle, morphology, surface modification, temperature and composition of its surrounding medium, etc.

A silver particle with a small particle size has a greater atomic density lattice face such as 111 lattice faces so that the silver particle has a higher activity, while the anti-cancer effect of the silver particle is closely related to its activity and its size. So, for the cancer cell, the angstrom silver with a smaller size is more effective against the cancer cell than nano-silver in a same concentration.

The following is a test of a high purity elementary substance angstrom silver injection on both in vitro and in vivo experiment of a malignant tumor cell to prove that angstrom silver material can effectively kill the cancer cell.

A first exemplary embodiment is shown a detection of the angstrom silver on a proliferation influence of human non-small cell lung cancer cells A549 and human hepatocellular carcinoma cells HepG2.

Reagents and consumables shown below: 1. HepG2, provided by HD Biosciences (Shanghai) Co., Ltd., and passed a mycoplasma detection; 2, F12K culture medium, American Gibco, Art. No. : 21127-022; 3. EMEM culture medium, American ATCC, Art. No. : 30-2003; 4. Fetal bovine serum (FBS), American Hyclone, Art. No. : CH30160.03; 5. Penicillin-streptomycin liquid, American Invitrogen, Art. No. : CH15140-122; 6. DG-5 (25 Ang) and DG-5 solvents, DG-6 (25 Ang) and DG-6 solvents respectively provided by Shenzhen AMCAN Medical Biotechnology Limited; 7. STSP, UK Tocris, Cat#3259; 8. DMSO, American Sigma, Art. No. : D4540; 9. 96-well cell culture plate, American Corning, Art. No. : 3610; 10. CellTiter-Glo Luminescent Cell Viability Assay, American Promega, Art. No. : G7571; 11. Plate reader, American Perkin Elmer, EnVision Multilabel Plate Reader.

Cell culture medium: 1. A549 culture medium: including 10 % fetal bovine serum and F12K culture medium with 100 U penicillin and 100 μg/mL streptomycin; 2. HepG2 culture medium: including 10 % fetal bovine serum and EMEM culture medium with 100 U penicillin and 100 μg/mL streptomycin.

An experimental method is shown below:

Compound preparation includes the following below:

1. The compounds DG-5 and DG-6 needed to be tested were diluted to a series of gradient concentration solutions using DG-5 and DG-6 solvents in a sterile condition, while the series of concentrations include 300ppm, 30ppm, 3ppm, 0.3ppm and 0.03ppm.

2. A positive chemical STSP is diluted to a series of concentration gradient solutions using a DMSO in the sterile condition, while the concentrations include 200 μM, 20 μM, 2 μM, 0.2 μM and 0.02 μM.

3. Only before starting an experiment, in the sterile condition, the prepared dg-5 gradient concentration solution is diluted 2 times by full cell culture medium. At this time, the gradient concentration of a compound to be measured includes 150ppm, 15ppm, 1.5ppm, 0.15ppm and 0.015ppm, which is a 2-by-compound solution to be used to treat cells.

4. Only before starting the experiment, in the sterile condition, the prepared dg-5 gradient concentration solution was diluted 100 times by the full cell culture medium. At this time, the gradient concentration of the compound to be measured includes 2 $\mu$M, 200 nM, 20 nM, 2 nM and 0.2 nM, which is a 2-by-compound solution to be used to treat cells.

Cytotoxicity test design

| | 1 | 2 | 3 | 4 | 5 | 6 | 7 | 8 | 9 | 10 | 11 | 12 |
|---|---|---|---|---|---|---|---|---|---|---|---|---|
| A | | | | | | | | | | | | |
| B | | DG-5(75 ppm) | | DG6-(75 ppm ) | | STSP(1uM ) | | DG-5 solvent | | 0.5%DMSO | 0.5%DMSO | |
| C | | DG-5(7.5 ppm) | | DG6-(7.5 ppm ) | | STSP(100nM ) | | | | | | |
| D | | DG-5(0.75 ppm) | | DG-6(0.75 ppm) | | STSP(10nM ) | | | | | | |
| E | | DG-5(0.075 ppm) | | DG-6(0.075 ppm) | | STSP(1nM ) | | DG-6 solvent | | | | |
| F | | DG-5(0.0075 ppm) | | DG-6(0.0075 ppm ) | | STSP(0.1n M) | | | | | | |
| G | | Cell | | | | | | | | | | |
| H | | | | | | | | | | | | |

HepG2

6000 cells in good conditions: HepG2
Day 0, 100 ul cells inoculated; Day 1, 100 ul compound inoculated

Culture Medium

Incubation time of the compound of 72 hours

Use of a Cell Titer GIO readout after 72 hours

|  | 1 | 2 | 3 | 4 | 5 | 6 | 7 | 8 | 9 | 10 | 11 | 12 |
|---|---|---|---|---|---|---|---|---|---|---|---|---|
| A |  |  |  |  |  |  |  |  |  |  |  |  |
| B |  | DG-5(75 ppm) | | DG6-(75 ppm ) | | STSP(1uM ) | | DG-5 solvent | | 0.5%DMSO | 0.5%DMSO | |
| C |  | DG-5(7.5 ppm) | | DG6-(7.5 ppm ) | | STSP(100n M ) | | | | | | |
| D |  | DG-5(0.75 ppm) | | DG-6(0.75 ppm) | | STSP(10nM ) | | | | | | |
| E |  | DG-5(0.075 ppm) | | DG-6(0.075 ppm) | | STSP(1nM ) | | DG-6 solvent | | | | |
| F |  | DG-5(0.0075 ppm) | | DG-6(0.0075 ppm ) | | STSP(0.1n M) | | | | | | |
| G |  | Cell | | | | | | | | | | |
| H |  |  |  |  |  |  |  |  |  |  |  |  |

A549

Culture Medium

5000 cells in good conditions: A549

Day 0, 100 ul cells inoculated; Day 1, 100 ul compound inoculated

Incubation time of the compound of 72 hours

Use of a Cell Titer GIO readout after 72 hours

Operation steps include the following:

1. The cell is inoculated in a 96-well cell culture plate the day before the treatment of the compound, while an inoculation density is shown in the experimental design above, and an inoculation volume was 100μL per hole.

2. The next day, the prepared 2-by-compound solution is added to the 96-well cell culture plate according to an arrangement diagram of the compound, and each hole is added to 100μL.

3. Gently oscillating the 96-well cell culture plate and placing it in a 37°C incubator to continue cultivating for 72 hours.

4. After reaching a designated incubation time, a prepared reagent is added to the 96-well cell culture plate according to a CellTiterGlo's reagent specification, and then it is fully blended under a room temperature, finally avoid light incubation for 10 minutes.

5. Placing the 96-well cell culture plate in a plate reader to analyze it, and setting it to read chemical luminescence and record data.

Data processing:

Readout in each hole needs to be converted to a cell viability. The cell viability can be calculated by the following formula:

$$\text{Cell viability} \ (\%) = \frac{\text{Sample hole readout}}{\text{Solvent control hole}} \times 100\%$$

The post-processing data will be used to perform nonlinear regression analysis using a GraphPad Prism 5 analysis software to obtain a dose-effect curve, at the same time, a half-killing concentration to the cell is obtained by calculating the compounds DG-5, DG-6 to be measured and the positive compound STSP.

An experimental result is shown below:

Original data is shown below:

After 3 days of HepG2 cell inoculation, the original data is shown below (the number in the sheet below is corresponding to the number of the cell in a region).

|   | 1 | 2 | 3 | 4 | 5 | 6 | 7 | 8 | 9 | 10 | 11 | 12 |
|---|---|---|---|---|---|---|---|---|---|---|---|---|
| A |  |  |  |  |  |  |  |  |  |  |  |  |
| B |  | 1900 | 2480 | 4060 | 5280 | 39260 | 45360 | 451040 | 453280 | 406420 | 382380 |  |
| C |  | 7540 | 8460 | 67700 | 91540 | 70380 | 89420 | 427480 | 399160 | 397900 | 397940 |  |
| D |  | 224860 | 192140 | 254200 | 262160 | 329340 | 316740 | 380280 | 390500 | 392640 | 404560 |  |
| E |  | 446620 | 388580 | 251920 | 253120 | 356480 | 347620 | 253280 | 270720 | 408980 | 404860 |  |
| F |  | 438620 | 448900 | 311780 | 294600 | 439500 | 416840 | 277700 | 287600 | 402600 | 415400 |  |
| G |  | 407420 | 412560 | 411840 | 419120 | 423920 | 407880 | 297760 | 283860 | 423080 | 402960 |  |
| H |  |  |  |  |  |  |  |  |  |  |  |  |

After 3 days of A549 cell inoculation, the original data is shown below (the number in the sheet below is corresponding to the number of the cell in the region).

|   | 1 | 2 | 3 | 4 | 5 | 6 | 7 | 8 | 9 | 10 | 11 | 12 |
|---|---|---|---|---|---|---|---|---|---|---|---|---|
| A |  |  |  |  |  |  |  |  |  |  |  |  |
| B |  | 10400 | 13040 | 279880 | 256400 | 69000 | 73660 | 665360 | 687560 | 616080 | 595520 |  |
| C |  | 246500 | 238180 | 534020 | 534680 | 173360 | 190180 | 670080 | 648640 | 599660 | 580040 |  |
| D |  | 600680 | 658360 | 601580 | 588160 | 418760 | 416940 | 668600 | 681560 | 630820 | 603220 |  |
| E |  | 659440 | 660960 | 607540 | 609740 | 607340 | 603900 | 596580 | 578200 | 602460 | 631820 |  |
| F |  | 605220 | 630180 | 763500 | 716680 | 758540 | 772520 | 626160 | 64086 0 | 663360 | 604620 |  |
| G |  | 634360 | 658020 | 675100 | 671500 | 662900 | 659940 | 565800 | 558560 | 599000 | 577820 |  |
| H |  |  |  |  |  |  |  |  |  |  |  |  |

The cell viability is seen below:

Percentage of cell growth: % HegG2 after compounds added

| Compound concentration (ppm) | Log [ppm] | DG-5-HepG2 | | DG-6-HepG2 | |
|---|---|---|---|---|---|
| 75 | 1.88 | 0.5 | 0.6 | 1.5 | 1.9 |
| 7.5 | 0.88 | 1.8 | 2.0 | 24.3 | 32.9 |
| 0.75 | -0.12 | 53.9 | 46.1 | 91.3 | 94.1 |
| 0.075 | -1.12 | 107.1 | 93.2 | 90.5 | 90.9 |
| 0.0075 | -2.12 | 105.2 | 107.7 | 112.0 | 105.8 |
| Control group concentration (nM) | Log[M] | STSP-HepG2 | | | |
| 1000 | -6.00 | 9.7 | 11.2 | | |
| 100 | -7.00 | 17.5 | 22.2 | | |
| 10 | -8.00 | 81.7 | 78.5 | | |
| 1 | -9.00 | 88.4 | 86.2 | | |
| 0.1 | -10.00 | 109.0 | 103.4 | | |

Percentage of cell growth: % A549 after joining compounds

| Compound concentration (ppm) | Log [ppm] | DG-5-A549 | | DG-6-A549 | |
|---|---|---|---|---|---|
| 75 | 1.88 | 1.6 | 1.9 | 47.1 | 43.1 |
| 7.5 | 0.88 | 36.8 | 35.5 | 89.8 | 90.0 |
| 0.75 | -0.12 | 89.6 | 98.2 | 101.2 | 99.0 |
| 0.075 | -1.12 | 98.4 | 98.6 | 102.2 | 102.6 |
| 0.0075 | -2.12 | 90.3 | 94.0 | 128.5 | 120.6 |
| Control group concentration (nM) | Log[M] | STSP-A549 | | | |
| 1000 | -6.00 | 11.3 | 12.1 | | |
| 100 | -7.00 | 28.5 | 31.2 | | |
| 10 | -8.00 | 68.8 | 68.5 | | |
| 1 | -9.00 | 99.8 | 99.2 | | |
| 0.1 | -10.00 | 124.6 | 126.9 | | |

A conclusion and discussion is shown below:

In the process of a cell vitality detection of nano-material DG-5 and DG-6, it is found that they showed a killing effect of the cell in both tumor cell lines HepG2 and A549. An IC50 value of the material DG-5 to the material HepG2 [(half maximal inhibitory concentration) is referred to a semi-inhibitory concentration of an antagonist that is measured, which it can indicate that a drug or substance (inhibitor) in inhibition of half amount of some biological programs (or substances contained in this program, such as enzyme, cell receptors or micro-organisms)] is 0.75 ppm, while An IC50 value of the material DG-6 to the material HepG2 is 4.03 ppm.

A second exemplary embodiment is shown below.

3D drug susceptibility detection result

| Serial No. | Sampling time | Name | Sex | Age | Disease | Experimenter |
|---|---|---|---|---|---|---|
| ① | November 12, 2015 | Chen * * | Male | 58 | Liver cancer | Xiong Feng |

| Technical system introduction: |
|---|
| The present center uses an only three-dimensional co-culture system in the world for detecting a variety of cells in vitro. The system includes two layers, a first layer is a gel layer of a matrix cell which is attached to a bottom of a culture dish, and a vascular endothelial cell and a tumor tissue cell are suspended in the gel layer; and a second layer is a cell culture liquid layer positioned on the top of the first layer. The system can simulate a whole process of tumor occurrence and tumor development, and the individualized treatment and drug resistance of cancer patients can be detected by observing regeneration level of the tumor cell and the vascular endothelial cell. |
| Detection of drug type: sorafenib, areca nut extract (ANE), rectum carcinoma drug, docetaxel, 50nm silver, 30nm silver, 25 Ang silver, pemetrexed, paclitaxel, 5-fluorouracil and carboplatin etc. |
| Detection and analysis result: Effective drugs: areca nut extract (ANE), rectum carcinoma drug, 25 Ang silver with best efficacy, docetaxel and paclitaxel with a second efficacy. Ineffective drugs: sorafenib, pemetrexed, 5-fluorouracil and carboplatin. |

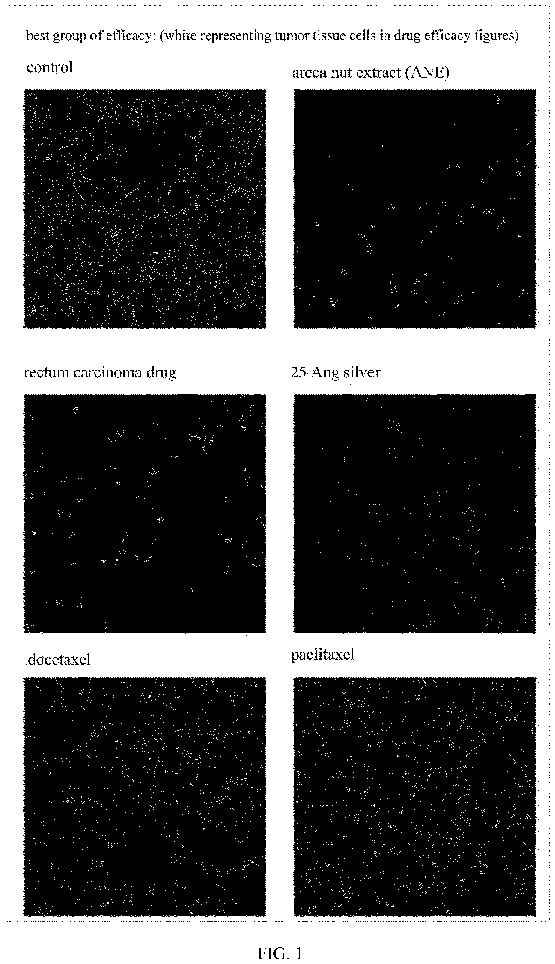

The best group of efficacy is shown in FIG. 1. (White representing tumor tissue cells in the drug efficacy figures)

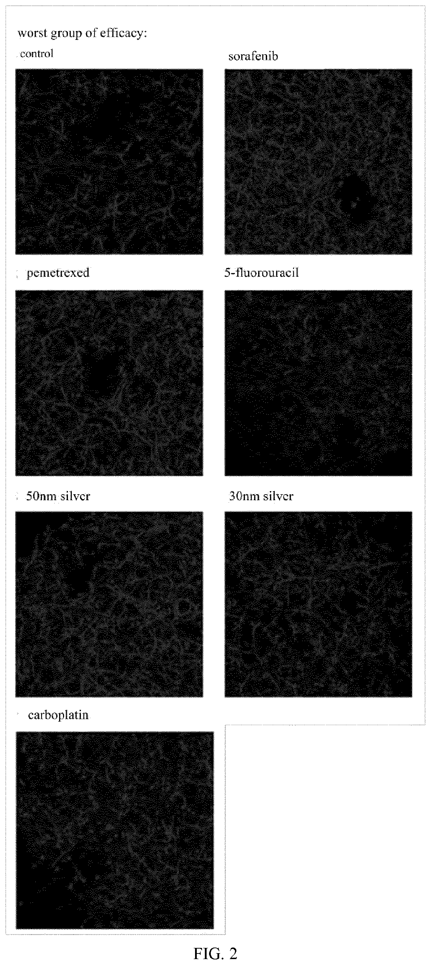

The worst group of efficacy is shown in FIG. 2.

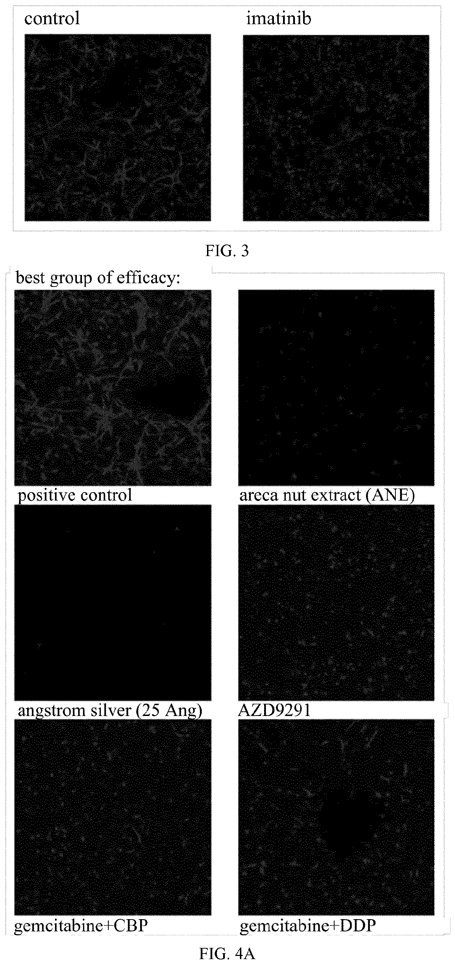

The effect of imatinib in the drug added is better shown in FIG. 3.

A third exemplary embodiment is shown below.

3D drug susceptibility detection result

| Serial No. | Sampling time | Name | Sex | Age | Disease | Experimenter |
|---|---|---|---|---|---|---|
| 0015 | Oct. 28, 2015 | Zhang * * | Male | 54 | NSCLC | Qing Miao, Luo |

| Technical system introduction: |
|---|
| The present center uses an only three-dimensional co-culture system in the world for detecting a variety of cells in vitro. The system includes two layers, a first layer is a gel layer of a matrix cell which is attached to a bottom of a culture dish, and a vascular endothelial cell and a tumor tissue cell are suspended in the gel layer; and a second layer is a cell culture liquid layer positioned on the top of the first layer. The system can simulate a whole process of tumor occurrence and tumor development, and the individualized treatment and drug resistance of cancer patients can be detected by observing regeneration level of the tumor cell and the vascular endothelial cell. |

| Detection of drug type: |
|---|
| areca nut extract (ANE), nano-silver 1 (50nm), nano-silver 2 (30nm), angstrom silver (25 Ang), erlotinib, gefitinib, AZD9291, escozul 1 (1:100), escozul 1 (1:500), escozul 1 (1:1000), cisplatin, carboplatin, irinotecan, gemcitabine, 5-Fu and pemetrexed. |

| Detection and analysis result: |
|---|
| The best group of efficacy: AZD9291, areca nut extract (ANE), angstrom silver (25 Ang), gemcitabine alone, gemcitabine+DDP, gemcitabine+CBP, irinotecan+DDP, irinotecan+CBP, pemetrexed+DDP, pemetrexed+CBP. Due to have a very poor efficacy by using permetrexed alone, thereby it is not recommended to use pemetrexed +DDP and pemetrexed +CBP. |
| The worst group of efficacy: escozul 1 (1:100), escozul 1 (1:500), escozul 1 (1:1000), nano-silver 1 (50nm), nano-silver 2 (30nm), erlotinib, gefitinib, 5-Fu and pemetrexed alone. |

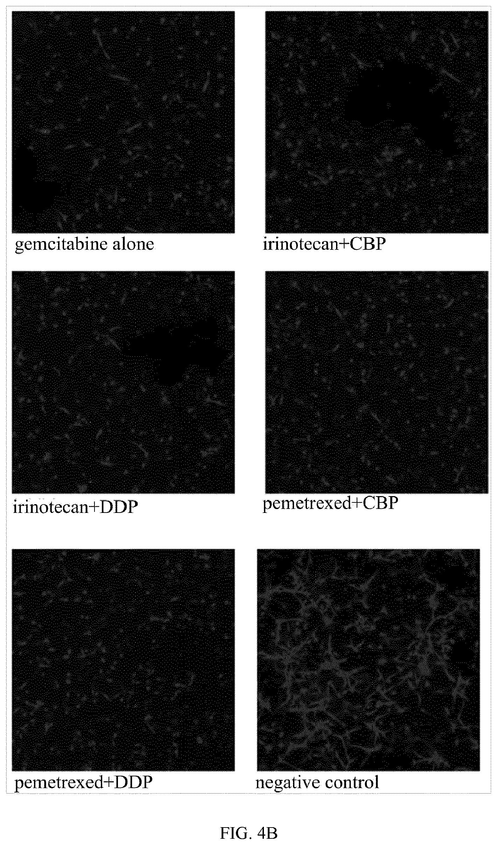

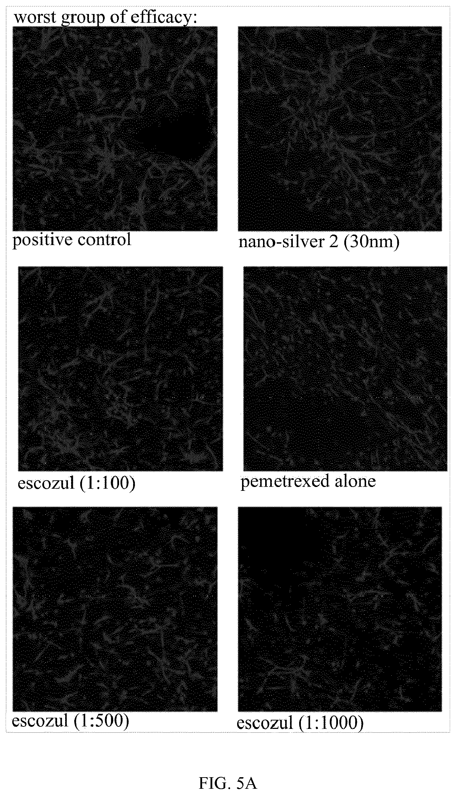

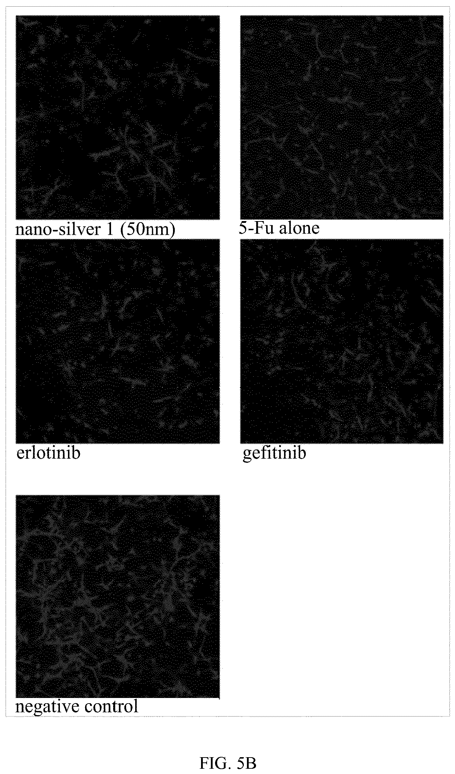

The best group of efficacy is shown in FIGS. 4A-4B and the worst group of efficacy is shown in FIGS. 5A-5B.

A fourth exemplary embodiment is shown below.

3D drug susceptibility detection result

| Serial No. | Sampling time | Name | Sex | Age | Disease | Experimenter |
|---|---|---|---|---|---|---|
| 0018 | Nov. 04, 2015 | Zhao * * | Male | 54 | Lung Tumor | Qing Miao, Luo |

| Technical system introduction: |
|---|
| The present center uses an only three-dimensional co-culture system in the world for detecting a variety of cells in vitro. The system includes two layers, a first layer is a gel layer of a matrix cell which is attached to a bottom of a culture dish, and a vascular endothelial cell and a tumor tissue cell are suspended in the gel layer; and a second layer is a cell culture liquid layer positioned on the top of the first layer. The system can simulate a whole process of tumor occurrence and tumor development, and the individualized treatment and drug resistance of cancer patients can be detected by observing regeneration level of the tumor cell and the vascular endothelial cell. |

| Detection of drug type: |
|---|
| areca nut extract (ANE), nano-silver 1 (50nm), nano-silver 2 (30nm), angstrom silver (25 Ang), erlotinib, gefitinib, AZD9291, escozul 1 (1:100), escozul 1 (1:500), escozul 1 (1:1000), cisplatin, carboplatin, irinotecan, gemcitabine, 5-Fu, pemetrexed, etoposide and vinoreline. |

| Detection and analysis result: |
|---|
| The best group of efficacy: areca nut extract (ANE), angstrom silver (25 Ang), nano-silver 2 (30nm), AZD9291, vinoreline+DDP, vinoreline+CBP, etoposide+DDP, etoposide+CBP, gemcitabine+DDP, gemcitabine+CBP, vinoreline alone + gemcitabine alone. The worst group of efficacy: nano-silver 1 (50nm), erlotinib, escozul 1 (1:100), escozul 1 (1:500), escozul 1 (1:1000), gefitinib, pemetrexed alone, 5-Fu alone, 5-Fu+CBP and 5-Fu+DDP. |

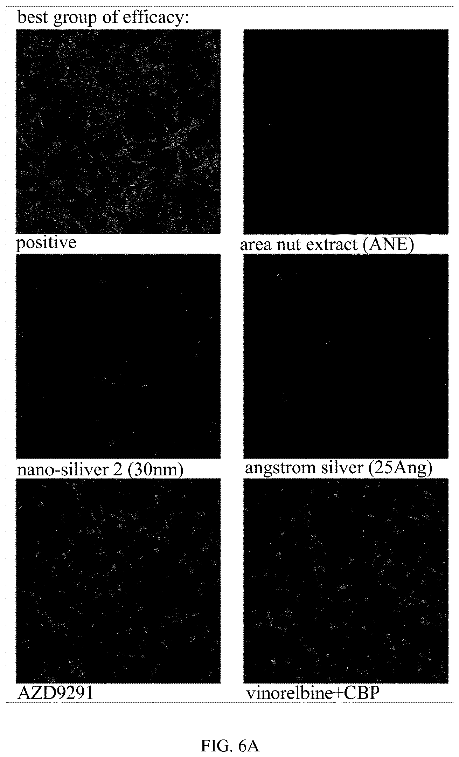

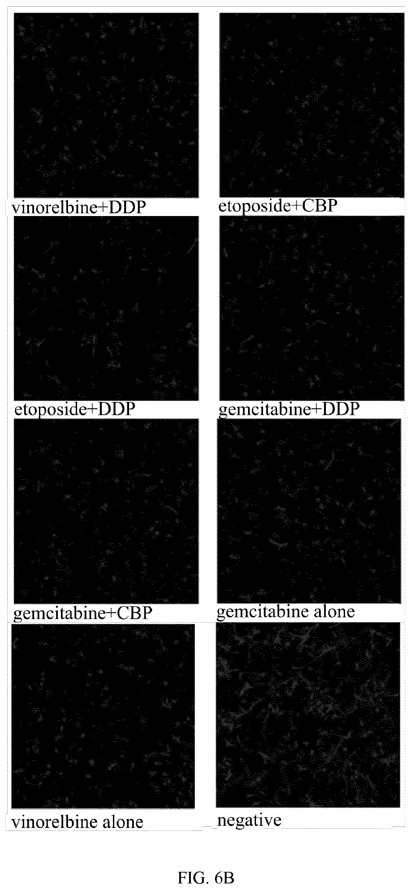

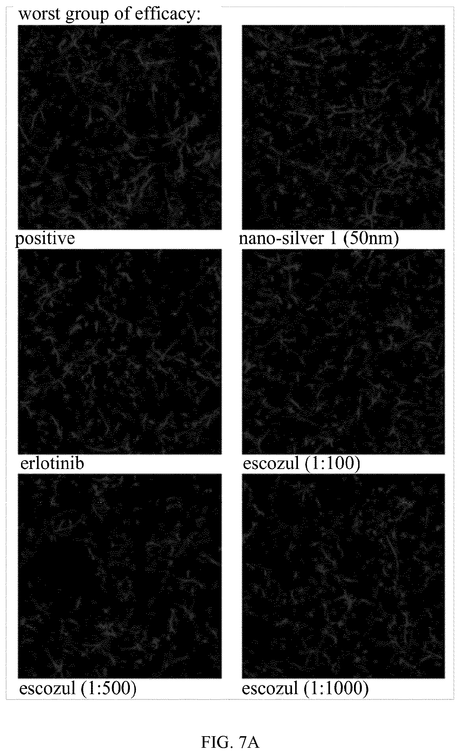

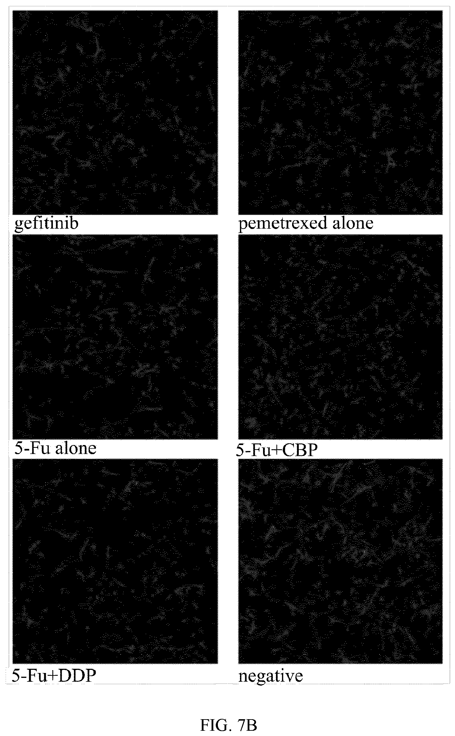

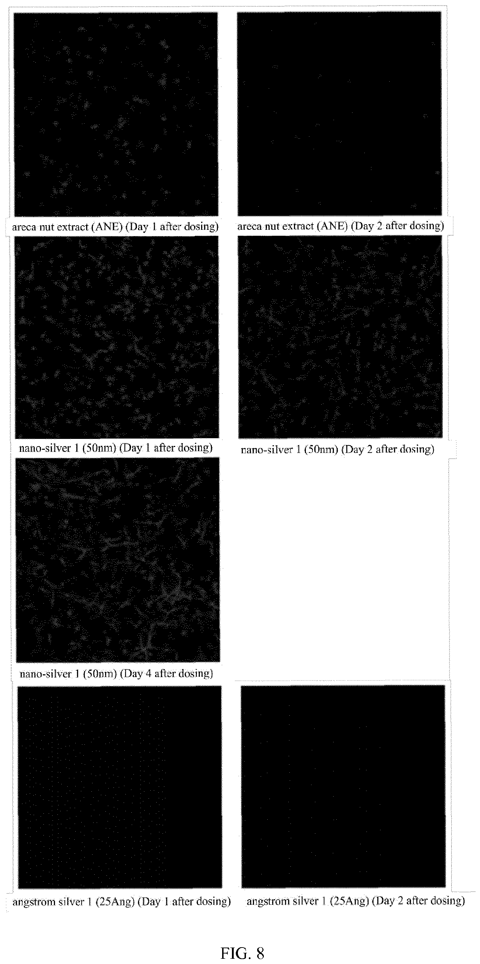

The best group of efficacy is shown in FIGS. 6A-6B, the worst group of efficacy is shown in FIGS. 7A-7B, and a group of new drug efficacy is shown in FIG. 8.

A fifth exemplary embodiment is shown below.

3D drug susceptibility detection result

| Serial No. | Sampling time | Name | Sex | Age | Disease | Experimenter |
|---|---|---|---|---|---|---|
| 0020 | Nov. 10, 2015 | Liu * * | Male | 75 | Lung Tumor pending | Huang Ping Qing Miao, Luo |

| Technical system introduction: |
|---|
| The present center uses an only three-dimensional co-culture system in the world for detecting a variety of cells in vitro. The system includes two layers, a first layer is a gel layer of a matrix cell which is attached to a bottom of a culture dish, and a vascular endothelial cell and a tumor tissue cell are suspended in the gel layer; and a second layer is a cell culture liquid layer positioned on the top of the first layer. The system can simulate a whole process of tumor occurrence and tumor development, and the individualized treatment and drug resistance of cancer patients can be detected by observing regeneration level of the tumor cell and the vascular endothelial cell. |

| Detection of drug type: |
|---|
| nano-silver (30nm), angstrom silver (25 Ang), AZD9291, escozul 1 (1:100), escozul 1 (1:500), escozul 1 (1:1000), irinotecan, gemcitabine, 5-Fu, pemetrexed, etoposide, vinoreline and endostar. |

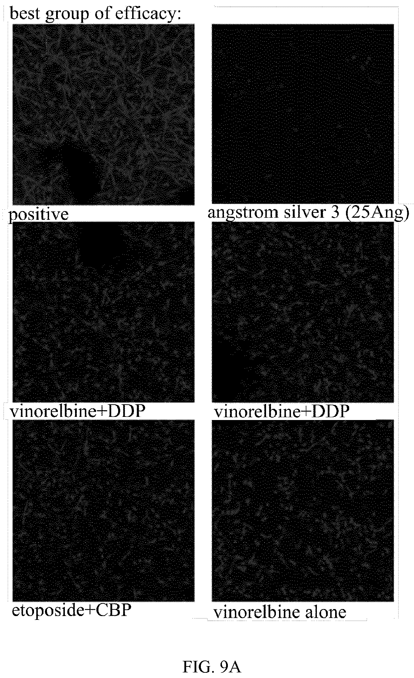

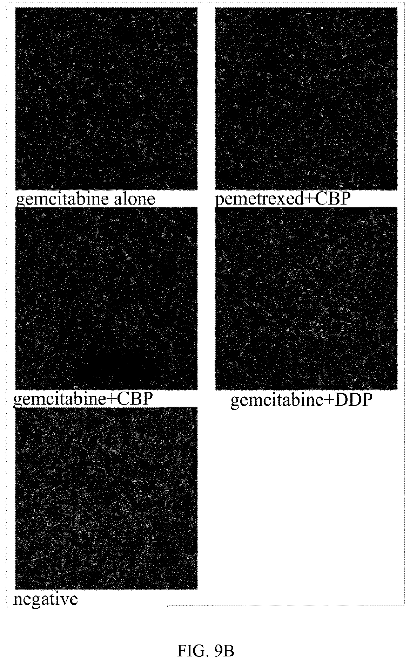

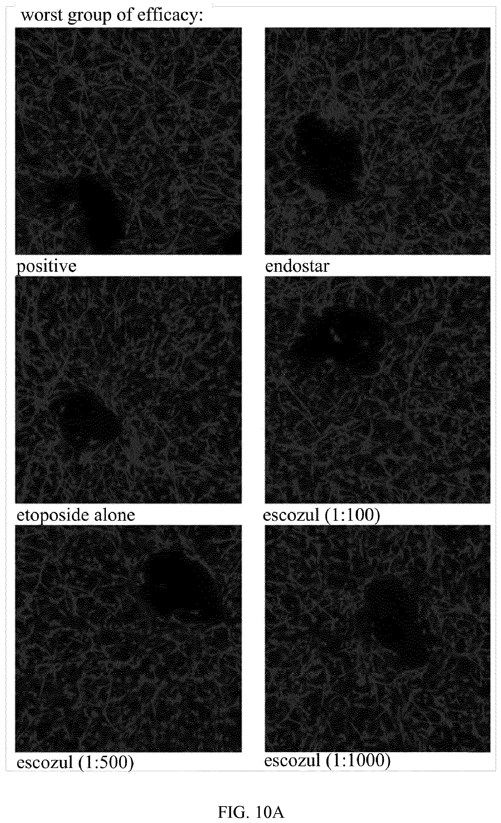

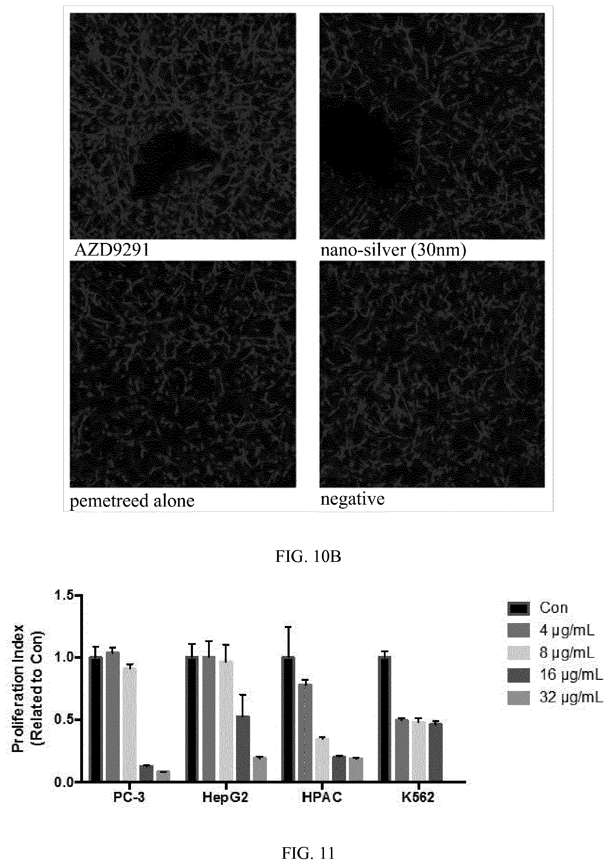

The best group of efficacy is shown in FIGS. 9A-9B, the worst group of efficacy is shown in FIGS. 10A-10B.

A sixth exemplary embodiment is shown below. A cytology experiment on human prostate cancer, liver cancer, pancreatic acinar epithelial cancer and leukemia is tested by the angstrom silver injection for the cancer inhibition in an experimental institution of Xiangya Hospital.

The experimental steps include the following below. 1. The experimental object is selected from cells belonging to human such as prostate cancer cells (PC-3), liver cancer cells (HegG2), pancreatic epithelial cells (HPAC) and chronic myeloid leukemia cells (K562); 2. The above cells arranged in a 5-by-10 manner are respectively inoculated in the 96-well cell culture plate, and after 24 hours, the cells are respectively interposed for 1-2 days by the angstrom silver injection with marked concentration; 3. A CCK8 kit is used to detect the effects of different concentrations of the angstrom silver on proliferation of the cancer cell in the experiments described above.

Referring to FIGS. 11-12, the experimental result includes the following:

FIG. 11 is shown a detection of cell proliferation activity of cancer cells interposed by the angstrom silver injection with marked concentration after 24 hours. In FIG. 11, Con represents a control group. PC-3 represents prostate cancer cells, HegG2 represents liver cancer cells, HPAC represents pancreatic epithelial cells, and K562 represents chronic myeloid leukemia cells, thereby a difference is significant compared with the control group, with * $p < 0.05$.

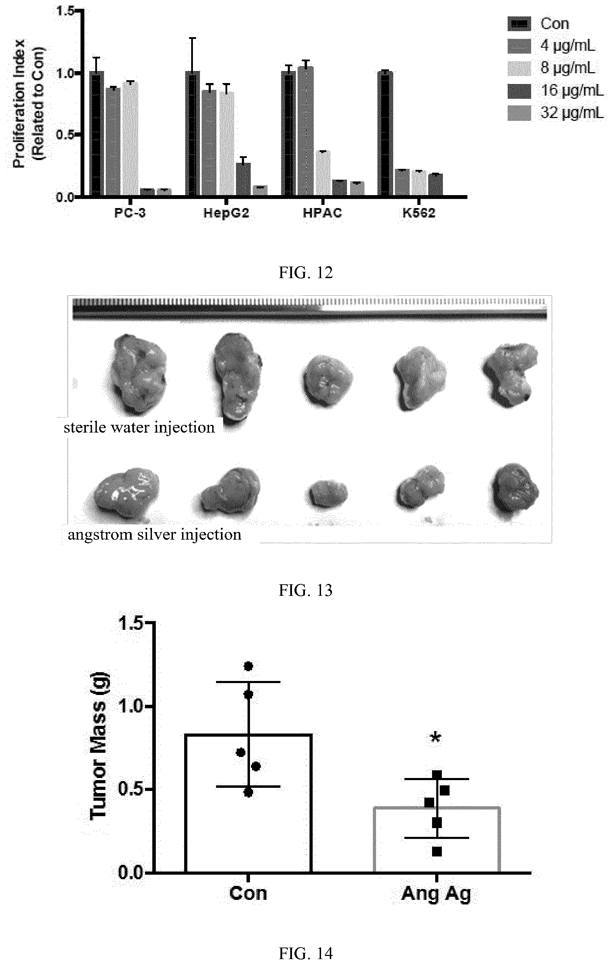

FIG. 12 is shown a detection of cell proliferation activity of cancer cells interposed by the angstrom silver injection with marked concentration after 48 hours. In FIG. 12, Con represents the control group. PC-3 represents prostate cancer cells, HegG2 represents liver cancer cells, HPAC represents pancreatic epithelial cells, and K562 represents chronic myeloid leukemia cells, thereby a difference is significant compared with the control group, with *$P < 0.05$.

A seventh exemplary embodiment is shown below. A cytology experiment on zoological pancreatic cancer is tested by the angstrom silver injection for the cancer inhibition in an experimental institution of Xiangya Hospital.

The experimental steps include the following below. 1. The experimental object is a thymless nude mouse, hereinafter referred as the nude mouse, which is 5 weeks old and weighs 17-20g; 2. 100 microliters pancreatic acinar epithelial cancer cells, which is equivalent to 106 humans' pancreatic acinar epithelial cancer cells (HPAC), are injected into a middle subcutaneous of an armpit of the nude mouse; 3. After 5 to 7 days, a subcutaneous tumor size is observed until the length of the tumor has reached 5 millimeters; 4. 150 microliters (75 microgram) angstrom silver injection is injected into a tail of the nude mouse by an intravenous injection way, and sterile water is used as control and drugs are administered for a next day; 5. After 30 days, the tumor is subcutaneously removed from the nude mouse, and the weight and volume of the tumor in the control group and the angstrom silver injection group are compared to each other.

Referring to FIGS. 13-15, the experimental result includes the following:

FIG. 13 is shown an HPAC tumor subcutaneously removed from the nude mouse.

FIG. 14 is shown a tumor mass of the HPAC tumor subcutaneously removed from the nude mouse. Con represents the control group. Ang Ag represents an angstrom silver injection intervention group, thereby a difference is significant compared with the control group, with *$P < 0.05$.

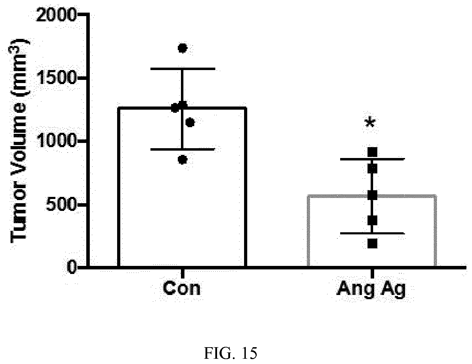

FIG. 15 is shown a tumor volume of the HPAC tumor subcutaneously removed from the nude mouse. Con represents the control group. Ang Ag represents the angstrom silver injection intervention group, thereby a difference is significant compared with the control group, with *$P < 0.05$.

Although the features and elements of the present disclosure are described as embodiments in particular combinations, each feature or element can be used alone or in other various combinations within the principles of the present disclosure to the full extent indicated by the broad general meaning of the terms in which the appended claims are expressed.

**Claims**

1. An angstrom silver injection for cancer inhibition which a composition of the angstrom silver injection comprising the following: angstrom-level elementary substance silver powder with a concentration between 0.005 g/L and 0.500 g/L, medicinal anhydrous fructose with a concentration between 0.5 g/L and 1.0 g/L, and sterile distilled water with a concentration between 998.500 g/L and 999.495 g/L.

2. The angstrom silver injection for cancer inhibition as claimed in claim 1, wherein purity of silver in the angstrom-level elementary substance silver powder is not less than 99.993%, and a particle size of silver particle is between 1 Ang and 25 Ang.

3. The angstrom silver injection for cancer inhibition as claimed in claim 2, wherein the shape of silver particle is spherical or oval.

4. A preparation method of an angstrom silver injection for a cancer inhibition comprising:

   S1, Obtaining high purity angstrom silver solution with a concentration between 0.005 g/L and 0.500 g/L by adding angstrom-level elementary substance silver powder, with its particle size between 1 Ang and 25 Ang, to sterile distilled water;
   S2, dispersing and then atomizing the angstrom silver solution by means of an ultrasonic device, and finally collecting it;
   S3, adding fructose in the collected angstrom silver solution, and dispersing the angstrom silver solution by the ultrasonic device once again, and finally left it standing;
   S4, Obtaining the angstrom silver injection by collecting atomization substance after the static angstrom silver solution atomized by means of the ultrasonic device.

5. The preparation method as claimed in claim 4, wherein the step of dispersing and then atomizing the angstrom silver solution by means of an ultrasonic device, and finally collecting it comprises: the angstrom silver solution is dispersed by an ultrasonic dispersion device with an ultrasonic power of 3 KW, a frequency between 30 KHZ and 40 KHZ and a dispersion time is 30-60 seconds; and then the angstrom silver solution is atomized by an ultrasonic atomization device with an ultrasonic power of 7.5 KW, a frequency between 15 KHZ and 20 KHZ and an atomization environment temperature in $2\pm5$ degree.

6. The preparation method as claimed in claim 4, wherein the step of adding fructose in the collected angstrom silver solution, and dispersing the angstrom silver solution by the ultrasonic device once again, and finally left it standing comprises: a concentration of the fructose is between 0.5 g/L and 1.0 g/L; when the ultrasonic dispersion is repeated, the ultrasonic power of the ultrasonic dispersion device is 0.5 KW, the frequency is 110-150 KHZ, the dispersion time is 10-15 seconds, and a static time is 15-30 minutes.

7. The preparation method as claimed in claim 4, wherein the step of obtaining the angstrom silver injection by collecting atomization substances after the static angstrom silver solution atomized by means of the ultrasonic device comprises the ultrasonic power of the ultrasonic atomization device is 7.5 KW, the frequency is 15-20 KHZ and the atomization environment temperature in $2\pm5$ degree.

8. An application of an angstrom silver injection comprising: the angstrom silver injection provided for a cancer inhibition with a composition of the angstrom silver injection comprising the following: angstrom-level elementary substance silver powder with a concentration between 0.005 g/L and 0.500 g/L, medicinal anhydrous fructose with a concentration between 0.5 g/L and 1.0 g/L, and sterile distilled water with a concentration between 998.500 g/L and 999.495 g/L.

9. The application of an angstrom silver injection as claimed in claim 8, wherein the purity of silver in the angstrom-level elementary substance silver powder is not less than 99.993%, and the particle size of silver particle is between 1 Ang and 25 Ang.

**10.** The application of an angstrom silver injection as claimed in claim 9, wherein the shape of silver particle is spherical or oval.

**11.** The application as claimed in claim 8, wherein the cancer is selected from lung cancer, liver cancer, pancreatic cancer, prostate cancer and leukemia.

best group of efficacy: (white representing tumor tissue cells in drug efficacy figures)

control

areca nut extract (ANE)

rectum carcinoma drug

25 Ang silver

docetaxel

paclitaxel

FIG. 1

worst group of efficacy:

control

sorafenib

pemetrexed

5-fluorouracil

50nm silver

30nm silver

carboplatin

FIG. 2

control | imatinib

FIG. 3

best group of efficacy:

positive control | areca nut extract (ANE)

angstrom silver (25 Ang) | AZD9291

gemcitabine+CBP | gemcitabine+DDP

FIG. 4A

gemcitabine alone

irinotecan+CBP

irinotecan+DDP

pemetrexed+CBP

pemetrexed+DDP

negative control

FIG. 4B

worst group of efficacy:

positive control

nano-silver 2 (30nm)

escozul (1:100)

pemetrexed alone

escozul (1:500)

escozul (1:1000)

FIG. 5A

nano-silver 1 (50nm)    5-Fu alone

erlotinib    gefitinib

negative control

FIG. 5B

best group of efficacy:

positive — area nut extract (ANE)

nano-siliver 2 (30nm) — angstrom silver (25Ang)

AZD9291 — vinorelbine+CBP

FIG. 6A

FIG. 6B

worst group of efficacy:

positive

nano-silver 1 (50nm)

erlotinib

escozul (1:100)

escozul (1:500)

escozul (1:1000)

FIG. 7A

FIG. 7B

areca nut extract (ANE) (Day 1 after dosing)

areca nut extract (ANE) (Day 2 after dosing)

nano-silver 1 (50nm) (Day 1 after dosing)

nano-silver 1 (50nm) (Day 2 after dosing)

nano-silver 1 (50nm) (Day 4 after dosing)

angstrom silver 1 (25Ang) (Day 1 after dosing)

angstrom silver 1 (25Ang) (Day 2 after dosing)

FIG. 8

FIG. 9A

gemcitabine alone

pemetrexed+CBP

gemcitabine+CBP

gemcitabine+DDP

negative

FIG. 9B

worst group of efficacy:

positive

endostar

etoposide alone

escozul (1:100)

escozul (1:500)

escozul (1:1000)

FIG. 10A

AZD9291

nano-silver (30nm)

pemetreed alone

negative

FIG. 10B

FIG. 11

FIG. 12

sterile water injection

angstrom silver injection

FIG. 13

FIG. 14

EP 3 511 009 A1

FIG. 15

29

Europäisches
Patentamt

European
Patent Office

Office européen
des brevets

# EUROPEAN SEARCH REPORT

Application Number

EP 18 18 0133

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/119818 A1 (LIU JINJUN [CN] ET AL) 4 May 2017 (2017-05-04) * abstract * * pages 2-3; examples 1-5 * * paragraph [0013]; claims * | 1-11 | INV. A61K33/38 A61K9/00 A61K47/26 A61P35/00 |
| Y | US 2017/266229 A1 (LIU JINJUN [CN] ET AL) 21 September 2017 (2017-09-21) * abstract * * paragraphs [0008], [0015] * * pages 2-3; examples 1-6 * * claims * | 1-11 | |
| Y | WO 2015/015301 A2 (PALAMA ILARIA EL [IT]; POLLINI MAURO [IT]; PALADINI FEDERICA [IT]; ACC) 5 February 2015 (2015-02-05) * abstract * * pages 11-19; examples 1-7 * * claims * | 1-11 | |
| Y | WO 2014/165968 A1 (NOBLE ADAM J [CA]; LACERDA ANDRESSA F [CA]) 16 October 2014 (2014-10-16) * abstract * * paragraphs [0086], [0087] * * paragraphs [0124] - [0145]; example 2 * * claims * | 1-11 | TECHNICAL FIELDS SEARCHED (IPC) A61K A61P |
| Y | CN 105 640 986 A (UNIV GUANGDONG TECHNOLOGY) 8 June 2016 (2016-06-08) * abstract * * claims * | 1-11 | |

-/--

The present search report has been drawn up for all claims

| Place of search | Date of completion of the search | Examiner |
|---|---|---|
| Munich | 22 January 2019 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 18 18 0133

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | V. Kondeti ET AL: "Ag+ reduction and silver nanoparticle synthesis at the plasma-liquid interface by an RF driven atmospheric pressure plasma jet: Mechanisms and the effect of surfactant", Journal of Vacuum Science & Technology A: Vacuum, Surfaces, and Films, 1 November 2017 (2017-11-01), page 61302, XP055531238, DOI: 10.1116/1.4995374 Retrieved from the Internet: URL:https://avs.scitation.org/doi/am-pdf/10.1116/1.4995374 * abstract * * page 2, last paragraph * ----- | 1-11 | |
| Y | JOHN C. HECKEL ET AL: "The effect of fructose derived carbon shells on the plasmon resonance and stability of silver nanoparticles", COLLOID & POLYMER SCIENCE, vol. 286, no. 13, 18 September 2008 (2008-09-18), pages 1545-1552, XP055532310, DE ISSN: 0303-402X, DOI: 10.1007/s00396-008-1929-4 * abstract * * page 1546, right-hand column, paragraph 1-2 * * page 1551, right-hand column, paragraph 1 * ----- | 1-11 | TECHNICAL FIELDS SEARCHED (IPC) |

The present search report has been drawn up for all claims

| Place of search | Date of completion of the search | Examiner |
|---|---|---|
| Munich | 22 January 2019 | Garabatos-Perera, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 18 0133

This annex lists the patent family members relating to the patent documents cited in the above-mentioned European search report.
The members are as contained in the European Patent Office EDP file on
The European Patent Office is in no way liable for these particulars which are merely given for the purpose of information.

22-01-2019

| Patent document cited in search report | | Publication date | Patent family member(s) | | Publication date |
|---|---|---|---|---|---|
| US 2017119818 | A1 | 04-05-2017 | CN<br>US<br>WO | 103933067 A<br>2017119818 A1<br>2015169103 A1 | 23-07-2014<br>04-05-2017<br>12-11-2015 |
| US 2017266229 | A1 | 21-09-2017 | CN<br>US<br>WO | 103908473 A<br>2017266229 A1<br>2015143928 A1 | 09-07-2014<br>21-09-2017<br>01-10-2015 |
| WO 2015015301 | A2 | 05-02-2015 | EP<br>US<br>WO | 3027281 A2<br>2016213711 A1<br>2015015301 A2 | 08-06-2016<br>28-07-2016<br>05-02-2015 |
| WO 2014165968 | A1 | 16-10-2014 | NONE | | |
| CN 105640986 | A | 08-06-2016 | NONE | | |

EPO FORM P0459

For more details about this annex : see Official Journal of the European Patent Office, No. 12/82